(19) Europäisches Patentamt
European Patent Office
Office européen des brevets

(11) **EP 4 692 786 A1**

(12) **EUROPEAN PATENT APPLICATION**
published in accordance with Art. 153(4) EPC

(43) Date of publication:
**11.02.2026 Bulletin 2026/07**

(21) Application number: **24780432.1**

(22) Date of filing: **27.03.2024**

(51) International Patent Classification (IPC):
***G01N 31/00*** (2006.01)   ***G01N 31/22*** (2006.01)

(52) Cooperative Patent Classification (CPC):
**G01N 31/00; G01N 31/22**

(86) International application number:
**PCT/JP2024/012224**

(87) International publication number:
**WO 2024/204326 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.03.2023 JP 2023057763**

(71) Applicant: **NOK Corporation
Minato-ku
Tokyo 105-8585 (JP)**

(72) Inventors:
• **NOGUCHI, Yuki
Fujisawa-shi, Kanagawa 251-0042 (JP)**
• **KOBAYASHI, Taiki
Fujisawa-shi, Kanagawa 251-0042 (JP)**
• **AOYAGI, Yuichi
Fujisawa-shi, Kanagawa 251-0042 (JP)**

(74) Representative: **Global IP Europe
Patentanwaltskanzlei
Pfarrstraße 14
80538 München (DE)**

(54) **HYDROGEN SULFIDE DETECTING AGENT**

(57)     A hydrogen sulfide detecting agent includes a polymer material that is a base, a coloring agent configured to develop a color by contact with hydrogen sulfide, and a color development accelerator configured to accelerate color development of the coloring agent.

FIG. 1

| | COMPARATIVE EXAMPLE A1 | EXAMPLE A1 | COMPARATIVE EXAMPLE A2 | EXAMPLE A2 |
|---|---|---|---|---|
| TYPES OF RUBBER | EPDM | EPDM | VMQ | VMQ |
| COLORING AGENTS | SILVER POWDER | SILVER POWDER | SILVER POWDER | SILVER POWDER |
| OIL | ABSENCE | SILICONE OIL | ABSENCE | SILICONE OIL |
| BEFORE EXPERIMENT | | | | |
| AFTER EXPERIMENT | | | | |

EP 4 692 786 A1

**Description**

TECHNICAL FIELD

This disclosure relates to techniques for detecting hydrogen sulfide.

BACKGROUND ART

Hydrogen sulfide is harmful to humans and corrodes or degrades metal and resin material. Thus, it is important to detect hydrogen sulfide. As a technique for detecting hydrogen sulfide, a sensor is used that applies a change in electrical characteristics or in thermal characteristics, caused by hydrogen sulfide being in contact with an element. However, to use the sensor, it is necessary to continuously supply electric power.

On the other hand, as a type in which electric power is not required, there is a technique for recognizing presence or absence of hydrogen sulfide by use of a material that changes color by contact with the hydrogen sulfide. For example, in Patent Document 1, a gas detector is proposed in which metal compounds for reacting with hydrogen sulfide to develop a color are dispersed in a base constituted of a polymer material. In addition, in Patent Document 2, a method is proposed in which by using a membrane that is permeable to hydrogen sulfide gas, concentration of detectable hydrogen sulfide gas can be controlled in accordance with permeability of the membrane.

Related Art Document

Patent Documents

Patent Document 1: Japanese Patent Application, Publication No. 2020-508434
Patent Document 2: Japanese Patent No. 4133640

SUMMARY OF THE INVENTION

Problem to be Solved by the Invention

However, to detect low concentrations of hydrogen sulfide by a technique disclosed in Patent Document 1 or in Patent Document 2, it is necessary to enhance the ability of a detector to perform detection. Thus, it is necessary to increase an amount of metal compounds; as a result, it is thought that the cost of producing the detector may increase. In view of the circumstances described above, an object of an aspect of this disclosure is to detect low concentrations of hydrogen sulfide and to control a coloring agent content.

Means for Solving Problem

To solve the above problems, a hydrogen sulfide detecting agent according to an aspect of this disclosure includes a polymer material that is a base, a coloring agent configured to develop a color by contact with hydrogen sulfide, and a color development accelerator configured to accelerate color development of the coloring agent.

BRIEF DESCRIPTION OF THE DRAWINGS

Fig. 1 shows a combination of materials included in each sample in experiment A, and photographs before and after color development thereof.
Fig. 2 shows a combination of materials included in each sample in experiment B, and color differences before and after color development thereof.

MODES FOR CARRYING OUT THE INVENTION

A hydrogen sulfide detecting agent according to an embodiment of this disclosure includes a coloring agent for developing a color by contact with hydrogen sulfide in a polymer material that is a base, and a color development accelerator for accelerating color development of the coloring agent.

2

The hydrogen sulfide detecting agent according to this embodiment is used to determine presence or absence of hydrogen sulfide. For example, in an electric vehicle provided with an all-solid-state battery including sulfide as a solid electrolyte, the hydrogen sulfide detecting agent according to this embodiment is disposed in a vicinity of the all-solid-state battery. When the solid electrolyte including the sulfide leaks from the all-solid-state battery, the leakage of the sulfide can be detected by color development of the hydrogen sulfide detecting agent.

A detection target for the hydrogen sulfide detecting agent according to this embodiment is, for example, hydrogen sulfide gas. Thus, the hydrogen sulfide detecting agent according to this embodiment can accurately detect whether a specific gas (gas) includes hydrogen sulfide. In addition, a concentration of hydrogen sulfide in the specific gas (a hydrogen sulfide content in the gas) can also be detected in accordance with a degree of color development of the hydrogen sulfide detecting agent. It should be noted that the detection target for the hydrogen sulfide detecting agent may be, for example, hydrogen sulfide dissolved in a liquid such as water.

Color Development Accelerator

The color development accelerator included in the hydrogen sulfide detecting agent according to this embodiment will be described.

At least some of the hydrogen sulfide in contact with the hydrogen sulfide detecting agent according to this embodiment is dissolved in the color development accelerator included in the hydrogen sulfide detecting agent. The inventors suppose that the hydrogen sulfide ($H_2S$) dissolved in the color development accelerator is ionized and reacts with the coloring agent after ionization. The inventors believe that according to the hydrogen sulfide detecting agent according to this embodiment, since most of the hydrogen sulfide is rapidly dissolved in the color development accelerator, color development easily occurs even when the coloring agent content is low. It should be noted that the expression "color development easily occurs" includes a sense that the coloring agent develops a color with high sensitivity even in a state in which the concentration of hydrogen sulfide is low, or a sense in which the coloring agent quickly develops a color by contact with hydrogen sulfide.

The type of color development accelerator is not particularly limited, and various types of oils may be given as examples of the color development accelerator, for example. By oil being added to the polymer material constituting the base, dissolution or permeation of hydrogen sulfide to the base is accelerated; as a result, hydrogen sulfide is likely to meet the coloring agent in the base. Thus, oil in which hydrogen sulfide can sufficiently dissolves is appropriate for the color development accelerator.

For example, low-molecular-weight oil may be given as an example of the color development accelerator. The low-molecular-weight oil is, for example, oil with a molecular weight of less than 10000. More preferably, oil with a molecular weight of 5000 or less (for example, 1000 or less) maybe given as an example of the color development accelerator.

In addition, a polar oil may be given as an example of the color development accelerator. The polar oil is oil with a molecule in which electrical polarities are unevenly distributed. Hydrogen sulfide, which is polar, is more easily dissolved in a polar oil than in the base made of a non-polar polymer material. Thus, according to a configuration in which a polar oil is used as the color development accelerator, it is possible to effectively accelerate color development by the coloring agent.

For example, a silicone oil, an ester oil, or a process oil may be given as an example of an oil used as the color development accelerator. The silicone oil is a non-polar oil. The ester oil is a polar oil. The process oil is a polar oil or a non-polar oil. The inventors found that according to a configuration in which the silicone oil, the ester oil, or the process oil is included in the color development accelerator, color development easily occurs even when the coloring agent content is low. It should be noted that a plurality of types of oils may be used as the color development accelerator. For example, a mixture of two or more oils selected from silicone oil, ester oil, and process oil may be used as the color development accelerator.

The color development accelerator content in the hydrogen sulfide detecting agent may be freely selected; for example, the color development accelerator content is 0.1 parts by mass or more and 30 parts by mass or less based on 100 parts by mass of the polymer material. If the color development accelerator content of the polymer material is too high, it is difficult to knead in a production process. On the other hand, if the color development accelerator content of the polymer material is too low, it is difficult for the coloring agent to develop a color. According to a configuration in which the color development accelerator content is 0.1 parts by mass or more and 30 parts by mass or less, it is possible to facilitate, at a high level, both kneading in the production process and color development of the coloring agent.

Coloring Agent

The coloring agent included in the hydrogen sulfide detecting agent according to this embodiment will be described. The coloring agent is an element or a compound, which develops a color by contact with hydrogen sulfide. For example, a chalcophile element, a siderophile element, or a compound thereof may be given as an example of the coloring agent.

For example, copper, zinc, gallium, germanium, arsenic, selenium, silver, cadmium, lanthanum, tin, antimony, tellurium, mercury, thallium, lead, bismuth, or polonium may be given as an example of the chalcophile element. For example, manganese, iron, cobalt, nickel, molybdenum, ruthenium, rhodium, palladium, rhenium, osmium, iridium, platinum, and gold may be given as examples of the siderophile element.

Among the materials indicated above, the coloring agent may be preferably at least one selected from the group of silver, silver compounds, copper, copper compounds, nickel, nickel compounds, cobalt, cobalt compounds, lead, lead compounds, iron, and iron compounds. More preferably, the coloring agent is constituted of at least one selected from the group of silver, silver compounds, lead, lead compounds, iron, and iron compounds. The inventors found that the hydrogen sulfide detecting agent using the coloring agent described above easily develops a color even when the coloring agent content is low.

It should be noted that the coloring agent may preferably be in powder form. However, the coloring agent may be in any form.

The coloring agent content of the hydrogen sulfide detecting agent is freely selected; for example, the coloring agent content is 10 parts by mass or more and 500 parts by mass or less based on 100 parts by mass of the polymer material. If the color development accelerator content of the polymer material is too high, it is difficult to knead in a production process. On the other hand, if the color development accelerator content of the polymer material is too low, it is difficult for the coloring agent to develop a color. According to a configuration in which the coloring agent content is 10 parts by mass or more and 500 parts by mass or less, it is possible to facilitate, at a high level, both kneading in the production process and color development of the coloring agent.

Polymer Material

The polymer material included in the hydrogen sulfide detecting agent according to this embodiment will be described.

The type of polymer material is not particularly limited, and any known polymer material may be used. For example, a rubber material such as a diene-based rubber, a hydrogenated product thereof, an olefin-based rubber, a halogen-containing rubber, a silicone rubber, a sulfur-containing rubber, and a fluoro-rubber may be given as examples of the polymer material of the hydrogen sulfide detecting agent.

Examples of the diene-based rubber and the hydrogenated product thereof include a natural rubber (NR), an isoprene rubber (IR), an epoxidized natural rubber, a styrene butadiene rubber (SBR), a butadiene rubber (BR) (a high cis BR and a low cis BR), an acrylonitrile butadiene rubber (NBR), a hydrogenated NBR, a hydrogenated SBR, etc.

An ethylene propylene rubber (EPM), an ethylene propylene diene rubber (EPDM), a maleic acid-modified ethylene propylene rubber (M-EPM), a maleic anhydride-modified ethylene-$\alpha$-olefin copolymer, an ethylene-glycidyl methacrylate copolymer, a maleic anhydride-modified ethylene-ethyl acrylate copolymer (modified EEA), a butyl rubber (IIR), a copolymer of isobutylene and either an aromatic vinyl or a diene-based monomer, an acrylic rubber (ACM), or ionomer, etc., may be given as an example of the olefin-based rubber.

A halogenated butyl rubber such as a brominated butyl rubber (Br-IIR) and a chlorinated butyl rubber (Cl-IIR), a halogenated isomonoolefin-p-alkylstyrene copolymer (for example, a brominated isobutylene-p-methylstyrene copolymer (BIMS)), a halogenated isobutylene-isoprene copolymer rubber, a chloroprene rubber (CR), an epichlorohydrin rubber (CHR), a chlorosulfonated polyethylene (CSM), a chlorinated polyethylene (CM), or a maleic acid-modified chlorinated polyethylene (M-CM), etc., may be given as an example of the halogen-containing rubber.

A methyl vinyl silicone rubber (VMQ), a dimethyl silicone rubber, or a methyl phenyl vinyl silicone rubber, etc., may be given as an example of the silicone rubber. A polysulfide rubber, etc., may be given as an example of the sulfur-containing rubber. A vinylidene fluoride-based rubber, a fluorine-containing vinyl ether-based rubber, a tetrafluoroethylene-propylene-based rubber, a fluorine-containing silicone-based rubber, or a fluorine-containing phosphazene-based rubber, etc., may be given as an example of the fluoro-rubber.

It should be noted that the polymer material may be preferably at least one selected from the group of ethylene propylene diene rubber (EPDM), silicone rubber, acrylic rubber, and fluoro-rubber.

The hydrogen sulfide detecting agent according to this embodiment includes the polymer material, the coloring agent, and the color development accelerator given above, and may include a different component. However, the different component content may be preferably 30% by mass or less, more preferably 15% by mass or less, still more preferably 5% by mass or less, and still more preferably 1% by mass or less.

Examples

Experiment A

A color development experiment (hereinafter referred to as "experiment A") was carried out on a plurality of samples (examples A1, A2, comparative examples A1, A2) prepared in the following combinations of materials. Specifically, mixed materials were thoroughly kneaded and then solidified into a disk to obtain a sample.

Example A1

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 130 parts by mass of silver powder (Silver powder FA-2-3 manufactured by DOWA ELECTRONICS MATERIALS CO., LTD.)
·Color development accelerator: 1 part by mass of silicone oil (TA-5 manufactured by Shin-Etsu Chemical Co., Ltd.)

Comparative Example A1

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 130 parts by mass of silver powder (Silver powder FA-2-3 manufactured by DOWA ELECTRONICS MATERIALS CO., LTD.)

In other words, the comparative example A1 is a sample in which the materials other than the color development accelerator (silicone oil) are the same as those of the example A1, the sample not including the color development accelerator of the example A1.

Example A2

·Polymer material: 100 parts by mass of silicone rubber (QP1-25 BASE JPN manufactured by SIR CORPORATION)
·Crosslinking agent: 2 parts by mass of peroxide crosslinking agent (TC-8 manufactured by Momentive Performance Materials Japan LLC)
·Coloring agent: 130 parts by mass of silver powder (Silver powder FA-2-3 manufactured by DOWA ELECTRONICS MATERIALS CO., LTD.)
·Color development accelerator: 1 part by mass of silicone oil (TA-5 manufactured by Shin-Etsu Chemical Co., Ltd.)

Comparative Example A2

·Polymer material: 100 parts by mass of silicone rubber (QP1-25 BASE JPN manufactured by SIR CORPORATION)
·Crosslinking agent: 2 parts by mass of peroxide crosslinking agent (TC-8 manufactured by Momentive Performance Materials Japan LLC)
·Coloring agent: 130 parts by mass of silver powder (Silver powder FA-2-3 manufactured by DOWA ELECTRONICS MATERIALS CO., LTD.)

In other words, the comparative example A2 is a sample in which the materials other than the color development accelerator (silicone oil) are the same as those of the example A2, the sample not including the color development accelerator of the example A2.

Experiment Method

A testing machine (model number: GH-180, manufactured by Yamazaki Seiki Laboratory Co., Ltd.) was used, and each of the samples was caused to be in contact with hydrogen sulfide gas under the following test conditions, and degree of change in color was observed.

Test Conditions

·Gas species: hydrogen sulfide
·Concentration: 5 ppm
·Temperature: 25 degrees Celsius
·Humidity: 75%
·Test duration: 24 hours

Results of Experiment A

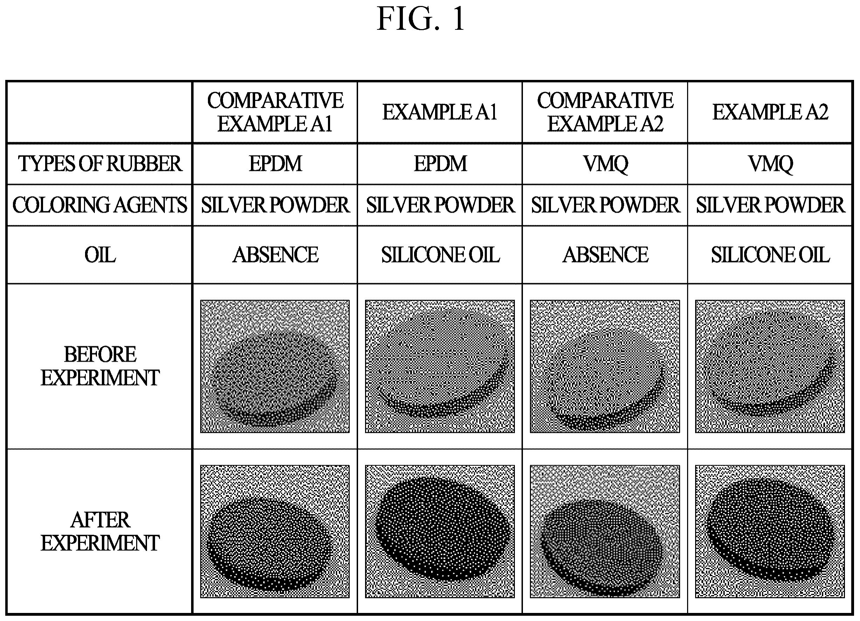

Fig. 1 shows photographs of each of the samples before and after contact with hydrogen sulfide gas. As shown in Fig. 1, the example A1 and the example A2 including the silicone oil were greater in degree of change in color than the comparative example A1 and the comparative example A2 including no silicone oil. In other words, it was conceived that in the example A1 and the example A2 corresponding to the hydrogen sulfide detecting agent according to this embodiment including the silicone oil, a degree of color development was increased compared to the comparative example A1 and the comparative example A2 including no silicone oil.

Experiment B

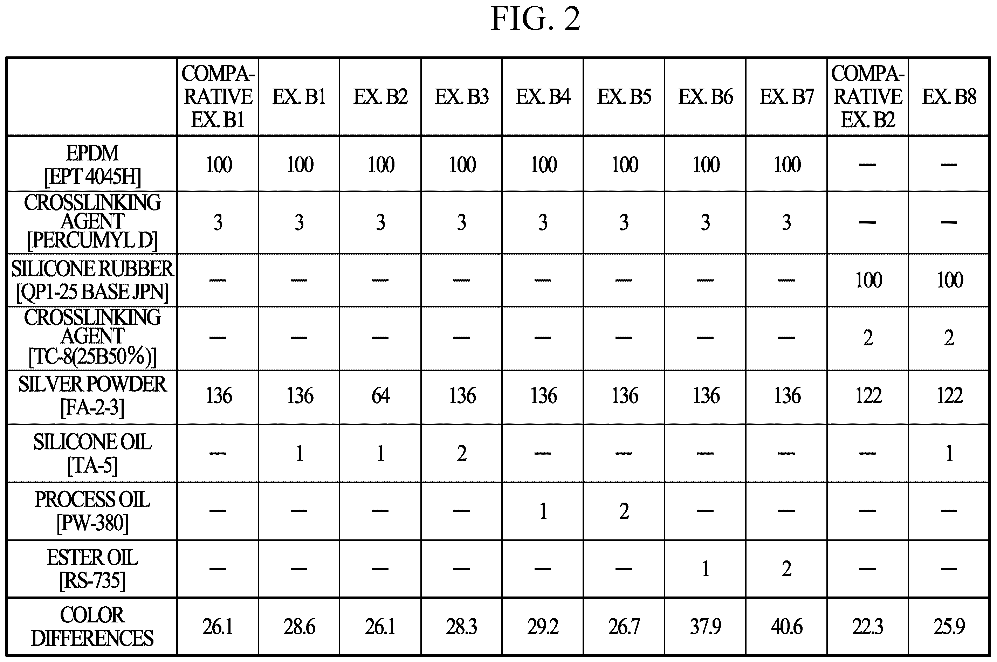

A color development experiment (hereinafter referred to as "experiment B") was carried out on a plurality of samples (examples B1 through B8, comparative examples B1 through B2) shown in Fig. 2. As in experiment A, mixed materials were thoroughly kneaded and then solidified into a disk to obtain a sample.

Example B1

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 136 parts by mass of silver powder (Silver powder FA-2-3 manufactured by DOWA ELECTRONICS MATERIALS CO., LTD.)
·Color development accelerator: 1 part by mass of silicone oil (TA-5 manufactured by Shin-Etsu Chemical Co., Ltd.)

Example B2

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 64 parts by mass of silver powder (Silver powder FA-2-3 manufactured by DOWA ELECTRONICS MATERIALS CO., LTD.)
·Color development accelerator: 1 part by mass of silicone oil (TA-5 manufactured by Shin-Etsu Chemical Co., Ltd.)

In other words, the example B2 is a sample in which the coloring agent content is reduced compared to the example B1.

Example B3

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 136 parts by mass of silver powder (Silver powder FA-2-3 manufactured by DOWA ELECTRONICS MATERIALS CO., LTD.)
·Color development accelerator: 2 parts by mass of silicone oil (TA-5 manufactured by Shin-Etsu Chemical Co., Ltd.)

In other words, the example B3 is a sample in which the color development accelerator content is increased compared to the example B1.

Example B4

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 136 parts by mass of silver powder (Silver powder FA-2-3 manufactured by DOWA ELECTRONICS MATERIALS CO., LTD.)
·Color development accelerator: 1 part by mass of process oil (PW-380 manufactured by Idemitsu Kosan Co., Ltd.)

In other words, the example B4 is a sample obtained by changing the type of color development accelerator from that of the example B1.

Example B5

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 136 parts by mass of silver powder (Silver powder FA-2-3 manufactured by DOWA ELECTRONICS MATERIALS CO., LTD.)
·Color development accelerator: 2 parts by mass of process oil (PW-380 manufactured by Idemitsu Kosan Co., Ltd.)

In other words, the example B5 is a sample in which the color development accelerator content is increased compared to the example B4.

Example B6

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 136 parts by mass of silver powder (Silver powder FA-2-3 manufactured by DOWA ELECTRONICS MATERIALS CO., LTD.)
·Color development accelerator: 1 part by mass of ester oil (TS-735 manufactured by ADEKA CORPORATION)

In other words, the example B6 is a sample obtained by changing the type of color development accelerator from that of example B1. It should be noted that the molecular weight of the ester oil in the example B6 is approximately 850.

Example B7

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 136 parts by mass of silver powder (Silver powder FA-2-3 manufactured by DOWA ELECTRONICS MATERIALS CO., LTD.)
·Color development accelerator: 2 parts by mass of ester oil (TS-735 manufactured by ADEKA CORPORATION)

In other words, the example B7 is a sample in which the color development accelerator content is increased compared to the example B4.

Example B8

·Polymer material: 100 parts by mass of silicone rubber (QP1-25 BASE JPN manufactured by SIR CORPORATION)
·Crosslinking agent: 2 parts by mass of peroxide crosslinking agent (TC-8 manufactured by Momentive Performance Materials Japan LLC)
·Coloring agent: 122 parts by mass of silver powder (Silver powder FA-2-3 manufactured by DOWA ELECTRONICS MATERIALS CO., LTD.)
·Color development accelerator: 1 part by mass of silicone oil (TA-5 manufactured by Shin-Etsu Chemical Co., Ltd.)

In other words, the example B8 is a sample obtained by changing types of polymer material and of cross-linking agent and the coloring agent content from those of the example B1.
Comparative Example B1

·Polymer material: 100 parts by mass of EPDM polymer (EPT4045 manufactured by Mitsui Chemicals, Inc.)
·Crosslinking agent: 3 parts by mass of peroxide crosslinking agent (PERCUMYL D manufactured by NOF CORPORATION)
·Coloring agent: 136 parts by mass of silver powder (Silver powder FA-2-3 manufactured by DOWA ELECTRONICS MATERIALS CO., LTD.)

In other words, the comparative example B1 is a sample obtained by excluding the color development accelerator from the example B1.

Comparative Example B2

·Polymer material: 100 parts by mass of silicone rubber (QP1-25 BASE JPN manufactured by SIR CORPORATION)
·Crosslinking agent: 2 parts by mass of peroxide crosslinking agent (TC-8 manufactured by Momentive Performance Materials Japan LLC)
·Coloring agent: 122 parts by mass of silver powder (Silver powder FA-2-3 manufactured by DOWA ELECTRONICS MATERIALS CO., LTD.)

In other words, the comparative example B1 is a sample obtained by excluding the color development accelerator from the example B1.
Each of the samples was caused to be in contact with hydrogen sulfide gas in a manner, and under conditions, similar to the experiment A. Fig. 2 shows a color difference ΔE*ab of each of the samples before and after contact with hydrogen sulfide gas. To measure the color difference ΔE*ab, a color difference meter (CR-400) manufactured by Konica Minolta Japan Co., Ltd was used.
A color difference ΔE*ab is a distance between chromaticity coordinates ($L_1^*$, $a_1^*$, $b_1^*$) of a sample before contact with hydrogen sulfide gas and chromaticity coordinates ($L_2^*$, $a_2^*$, $b_2^*$) of the sample after contact with the hydrogen sulfide gas. Each of the chromaticity coordinates ($L_n^*$, $a_n^*$, $b_n^*$) (n = 1, 2) are coordinates in the L*a*b* color space.

Specifically, the color difference ΔE*ab is represented by the following equation.

$$\Delta E^{*}ab = \{(L2^{*} - L1^{*})^{2} + (a2^{*} - a1^{*})^{2} + (a2^{*} - a1^{*})^{2}\}^{1/2}$$

In other words, it is possible to determine that the greater the color difference ΔE*ab, the greater a change in color of the sample before and after contact with hydrogen sulfide gas.

Results of Experiment B

As shown in Fig. 2, the color differences ΔE*ab of the examples B1 and B3 through B7 that include the color development accelerator were greater in numerical value than the color difference ΔE*ab of the comparative example B1 that includes no color development accelerator. Similarly, the color difference ΔE*ab of the example B8 was greater in numerical value than the color difference ΔE*ab of the comparative example B2. From the results described above, it was confirmed that the degree of color development was increased by adding the color development accelerator.

The color difference ΔE*ab of the example B2 was equal in numerical value to the color difference ΔE*ab of the comparative example B1. However, the coloring agent content of the example B2 was half of the coloring agent content of the comparative example B1. From the results described above, it was confirmed that it was possible to maintain the same color development by adding the color development accelerator when the coloring agent was reduced (for example, halved).

The color differences ΔE*ab of the examples B1 through B5 were greater than or equal to the color difference E*ab of the comparative example B1. From the results described above, it was confirmed that the degree of color development was increased by adding silicone oil or process oil as the color development accelerator. In addition, the color differences ΔE*ab of the examples B6 and B7 including the ester oil, in other words, polar oil as a color development accelerator were greater in numerical value than the color differences ΔE*ab of the examples B1 through B5. From the results described above, it was confirmed that the degree of color development was more effectively increased by using polar oil as the color development accelerator.

The color difference ΔE*ab of the example B7 was greater in numerical value than the color difference ΔE*ab of the example B6 in which the color development accelerator content was less than that of the example B7. From the results described above, it was confirmed that the degree of color development was more effectively increased by increasing the color development accelerator (in particular, polar oil) content.

The color difference ΔE*ab of the example B8 was greater in numerical value than the color difference E*ab of the comparative example B2. Thus, it was confirmed that the degree of color development was increased by adding the color development accelerator when a polymer material (silicone rubber) of a type different from those of the examples B1 through B7 was used as the base.

Supplementary Notes

The following configurations are derivable from the foregoing embodiment, for example.

A hydrogen sulfide detecting agent according to one aspect (first aspect) of this disclosure includes a polymer material that is a base, a coloring agent configured to develop a color by contact with hydrogen sulfide, and a color development accelerator configured to accelerate color development of the coloring agent. According to this aspect, it is possible to detect hydrogen sulfide with high accuracy. For example, it is possible to detect a low concentration of hydrogen sulfide and to control a coloring agent content.

In a specific example (second aspect) of the first aspect, the coloring agent is constituted of at least one selected from the group of silver, silver compounds, copper, copper compounds, nickel, nickel compounds, cobalt, cobalt compounds, lead, lead compounds, iron, and iron compounds.

In a specific example (third aspect) of the first aspect or the second aspect, the color development accelerator is an oil. In a specific example (fourth aspect), the color development accelerator is an oil with a molecular weight of 5000 or less. In a more preferred example (fifth aspect), the color development accelerator is a polar oil. According to the third aspect through the fifth aspect, it is possible to effectively accelerate color development by the coloring agent.

As a specific example of any one of the first aspect through the fifth aspect, for example, an example (sixth aspect) in which the color development accelerator includes a silicone oil, an example (seventh aspect) in which the color development accelerator includes an ester oil, or an example (eighth aspect) in which the color development accelerator includes a process oil is given. It should be noted that the color development accelerator may be constituted of a mixture of a plurality of types of materials (for example, oils).

In a specific example (ninth aspect) of any one of the first aspect through the eighth aspect, a content of the coloring agent is 10 parts by mass or more and 500 parts by mass or less based on 100 parts by mass of the polymer material. In addition, in a specific example (tenth aspect) of any one of the first aspect through the ninth aspect, a content of the color development accelerator is 0.1 parts by mass or more and 30 parts by mass or less based on 100 parts by mass of the polymer material. According to aspects described above, it is possible to facilitate, at a high level, both kneading in a production process and color development of the coloring agent.

It should be noted that in any one of the first aspect through the eighth aspect, a configuration in which the coloring agent content is 10 parts by mass or more, or a configuration in which the color development accelerator content is 0.1 parts by mass or more is assumed. According to this aspect, it is possible to maintain ease of color development of the coloring agent compared to a configuration in which the coloring agent or the color development accelerator is small. In addition, in any one of the first aspect through the eighth aspect, a configuration in which the coloring agent content is 500 parts by mass or less, or a configuration in which the color development accelerator content is 30 parts by mass or less is assumed. According to this aspect, it is possible to facilitate kneading the materials in the production process compared to a configuration in which the coloring agent or the color development accelerator is large.

**Claims**

1.  A hydrogen sulfide detecting agent comprising:

    a polymer material that is a base;
    a coloring agent configured to develop a color by contact with hydrogen sulfide; and
    a color development accelerator configured to accelerate color development of the coloring agent.

2.  The hydrogen sulfide detecting agent according to claim 1, wherein the coloring agent is constituted of at least one selected from a group of silver, silver compounds, copper, copper compounds, nickel, nickel compounds, cobalt, cobalt compounds, lead, lead compounds, iron, and iron compounds.

3.  The hydrogen sulfide detecting agent according to claim 1 or claim 2, wherein the color development accelerator is an oil.

4.  The hydrogen sulfide detecting agent according to claim 3, wherein the color development accelerator is an oil with a molecular weight of 5000 or less.

5.  The hydrogen sulfide detecting agent according to claim 3 or claim 4, wherein the color development accelerator is a polar oil.

6.  The hydrogen sulfide detecting agent according to claim 3, wherein the color development accelerator includes a silicone oil.

7.  The hydrogen sulfide detecting agent according to claim 3, wherein the color development accelerator includes an ester oil.

8.  The hydrogen sulfide detecting agent according to claim 3, wherein the color development accelerator includes a process oil.

9.  The hydrogen sulfide detecting agent according to claim 1, wherein a content of the coloring agent is 10 parts by mass or more and 500 parts by mass or less based on 100 parts by mass of the polymer material.

10. The hydrogen sulfide detecting agent according to claim 9, wherein a content of the color development accelerator is 0.1 parts by mass or more and 30 parts by mass or less based on 100 parts by mass of the polymer material.

# FIG. 1

|  | COMPARATIVE EXAMPLE A1 | EXAMPLE A1 | COMPARATIVE EXAMPLE A2 | EXAMPLE A2 |
|---|---|---|---|---|
| TYPES OF RUBBER | EPDM | EPDM | VMQ | VMQ |
| COLORING AGENTS | SILVER POWDER | SILVER POWDER | SILVER POWDER | SILVER POWDER |
| OIL | ABSENCE | SILICONE OIL | ABSENCE | SILICONE OIL |
| BEFORE EXPERIMENT | | | | |
| AFTER EXPERIMENT | | | | |

# FIG. 2

| | COMPA-RATIVE EX. B1 | EX. B1 | EX. B2 | EX. B3 | EX. B4 | EX. B5 | EX. B6 | EX. B7 | COMPA-RATIVE EX. B2 | EX. B8 |
|---|---|---|---|---|---|---|---|---|---|---|
| EPDM [EPT 4045H] | 100 | 100 | 100 | 100 | 100 | 100 | 100 | 100 | — | — |
| CROSSLINKING AGENT [PERCUMYL D] | 3 | 3 | 3 | 3 | 3 | 3 | 3 | 3 | — | — |
| SILICONE RUBBER [QP1-25 BASE JPN] | — | — | — | — | — | — | — | — | 100 | 100 |
| CROSSLINKING AGENT [TC-8(25B50%)] | — | — | — | — | — | — | — | — | 2 | 2 |
| SILVER POWDER [FA-2-3] | 136 | 136 | 64 | 136 | 136 | 136 | 136 | 136 | 122 | 122 |
| SILICONE OIL [TA-5] | — | 1 | 1 | 2 | — | — | — | — | — | 1 |
| PROCESS OIL [PW-380] | — | — | — | — | 1 | 2 | — | — | — | — |
| ESTER OIL [RS-735] | — | — | — | — | — | — | 1 | 2 | — | — |
| COLOR DIFFERENCES | 26.1 | 28.6 | 26.1 | 28.3 | 29.2 | 26.7 | 37.9 | 40.6 | 22.3 | 25.9 |

EP 4 692 786 A1

# EP 4 692 786 A1

| INTERNATIONAL SEARCH REPORT | International application No. |
| --- | --- |
| | **PCT/JP2024/012224** |

## A. CLASSIFICATION OF SUBJECT MATTER

***G01N 31/00***(2006.01)i; ***G01N 31/22***(2006.01)i
FI:   G01N31/00 P; G01N31/22 121A

According to International Patent Classification (IPC) or to both national classification and IPC

## B. FIELDS SEARCHED

Minimum documentation searched (classification system followed by classification symbols)

G01N31/00; G01N31/22

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
| --- | --- | --- |
| X | WO 2021/256087 A1 (MITSUBISHI ELECTRIC CORPORATION) 23 December 2021 (2021-12-23) paragraphs [0014], [0016], [0022], examples | 1-2, 9-10 |
| A | | 3-8 |
| X | JP 2006-343282 A (RIKEN KEIKI CO., LTD.) 21 December 2006 (2006-12-21) claims 1, 3, paragraphs [0006], [0009] | 1-2, 9-10 |
| A | JP 49-46276 B1 (DKK CORP.) 09 December 1974 (1974-12-09) table 1 | 1-10 |
| A | JP 3-142359 A (SHIN-KOBE ELECTRIC MACHINERY CO., LTD.) 18 June 1991 (1991-06-18) claims | 1-10 |
| A | JP 2005-37188 A (RIKEN KEIKI CO., LTD.) 10 February 2005 (2005-02-10) claims | 1-10 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **03 June 2024** | **11 June 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**EP 4 692 786 A1**

## INTERNATIONAL SEARCH REPORT

International application No.

**PCT/JP2024/012224**

### C. DOCUMENTS CONSIDERED TO BE RELEVANT

| Category* | Citation of document, with indication, where appropriate, of the relevant passages | Relevant to claim No. |
|---|---|---|
| A | WO 2023/017823 A1 (THE UNIV OF KITAKYUSHU) 16 February 2023 (2023-02-16) abstract | 1-10 |
| A | JP 2003-121366 A (RIKEN KEIKI CO., LTD.) 23 April 2003 (2003-04-23) abstract | 1-10 |
| A | JP 58-99753 A (MDA SCIENTIFIC, INC.) 14 June 1983 (1983-06-14) claims | 1-10 |
| A | JP 6-186166 A (RIKEN KEIKI CO., LTD.) 08 July 1994 (1994-07-08) abstract | 1-10 |
| A | JP 4044702 B2 (DAIICHI PURE CHEMICALS CO., LTD.) 06 February 2008 (2008-02-06) claims | 1-10 |
| A | JP 2020-508434 A (NITTO DENKO CORPORATION) 19 March 2020 (2020-03-19) claims | 1-10 |
| A | US 2011/0033943 A1 (TOTAL S.A.) 10 February 2011 (2011-02-10) claims | 1-10 |
| A | JP 2021-160975 A (TOYO SEIKAN GROUP HOLDINGS LTD.) 11 October 2021 (2021-10-11) abstract | 1-10 |

Form PCT/ISA/210 (second sheet) (July 2022)

14

## INTERNATIONAL SEARCH REPORT
### Information on patent family members

International application No.

**PCT/JP2024/012224**

| Patent document cited in search report | | | Publication date (day/month/year) | Patent family member(s) | | | Publication date (day/month/year) |
|---|---|---|---|---|---|---|---|
| WO | 2021/256087 | A1 | 23 December 2021 | (Family: none) | | | |
| JP | 2006-343282 | A | 21 December 2006 | (Family: none) | | | |
| JP | 49-46276 | B1 | 09 December 1974 | (Family: none) | | | |
| JP | 3-142359 | A | 18 June 1991 | (Family: none) | | | |
| JP | 2005-37188 | A | 10 February 2005 | (Family: none) | | | |
| WO | 2023/017823 | A1 | 16 February 2023 | JP | 2023-153800 | A | |
| JP | 2003-121366 | A | 23 April 2003 | (Family: none) | | | |
| JP | 58-99753 | A | 14 June 1983 | US claims | 4420567 | A | |
| | | | | GB | 2108662 | A | |
| | | | | DE | 3239092 | A | |
| | | | | CA | 1181330 | A | |
| JP | 6-186166 | A | 08 July 1994 | US abstract | 5397536 | A | |
| JP | 4044702 | B2 | 06 February 2008 | US claims | 6969613 | B1 | |
| | | | | WO | 2000/034771 | A1 | |
| | | | | EP | 1143244 | A1 | |
| | | | | TW | 546475 | B | |
| | | | | CN | 1329720 | A | |
| | | | | KR | 10-0639059 | B1 | |
| JP | 2020-508434 | A | 19 March 2020 | US claims | 2020/0003697 | A1 | |
| | | | | WO | 2018/152430 | A1 | |
| | | | | CN | 110291234 | A | |
| | | | | KR | 10-2019-0116300 | A | |
| US | 2011/0033943 | A1 | 10 February 2011 | US | 8986625 | B2 | |
| | | | | GB | 2471428 | A | |
| | | | | WO | 2009/130559 | A1 | |
| JP | 2021-160975 | A | 11 October 2021 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020508434 A

- JP 4133640 B